(19) Europäisches Patentamt
European Patent Office
Office européen des brevets

(11) **EP 4 567 408 A1**

(12) **EUROPEAN PATENT APPLICATION**
published in accordance with Art. 153(4) EPC

(43) Date of publication:
**11.06.2025 Bulletin 2025/24**

(21) Application number: **23849854.7**

(22) Date of filing: **12.07.2023**

(51) International Patent Classification (IPC):
**G01N 21/59** (2006.01)   **C09D 7/61** (2018.01)
**C09D 201/00** (2006.01)

(52) Cooperative Patent Classification (CPC):
**C09D 7/61; C09D 201/00; G01N 21/59**

(86) International application number:
**PCT/JP2023/025720**

(87) International publication number:
**WO 2024/029295 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.08.2022 JP 2022122763**

(71) Applicant: **Toyo Aluminium Kabushiki Kaisha
Osaka-shi, Osaka 541-0056 (JP)**

(72) Inventor: **HAYAMI Kazuki
Osaka-shi, Osaka 541-0056 (JP)**

(74) Representative: **Kador & Partner Part mbB
Corneliusstraße 15
80469 München (DE)**

(54) **HIDING POWER EVALUATION METHOD**

(57)    The method for evaluating hiding power is a method for evaluating hiding power of a particle containing body which contains particles of a hiding power evaluation target, the method including a step of calculating, on a basis of a transmission amount ($I$) of electromagnetic waves of the particle containing body and a base material and a transmission amount ($I_0$) of electromagnetic waves of a non-particle containing body and a base material, absorbance (Abs) of electromagnetic waves by a below-mentioned Equation (1), the non-particle containing body having same composition as composition of the particle containing body except that the non-particle containing body does not contain the particles of the hiding power evaluation target,

$$\text{Equation (1)} \quad \text{Abs} = -\log(I/I_0).$$

**FIG. 1**

EP 4 567 408 A1

**Description**

TECHNICAL FIELD

The present invention relates generally to a method for evaluating hiding power of a particle containing body which contains particles and, in particular, to a method for evaluating hiding power of coating film which uses pigment as particles.

BACKGROUND ART

Conventionally, hiding power of coating film which contains particles of pigment or the like is evaluated. For example, measurement of a degree at which a basic color is hidden by paint (hiding power) can be obtained by applying paint onto white and black undercoat and actually measuring a tristimulus value of a dried color card as in JIS K 5600-4-1: 1999K (ISO/FDIS 6504-3: 1998).

In addition, since metal effect pigment does not transmit light, it is considered that hiding power depends on an area of a surface of coating film, covered by the metal effect pigment. Therefore, a water surface diffusion area (WCA) prescribed in JIS K 5906: 1998 (Non-Patent Literature 2) is used as an index which represents hiding power of the coating film using the metal effect pigment.

The hiding power of the coating film is adjusted by repeating adjustment of a pigment concentration in the paint or a film thickness of the coating film and visual evaluation of the hiding power.

CITATION LIST

NON-PATENT LITERATURE

    Non-Patent Literature 1: JIS K 5600-4-1:1999K(ISO/FDIS 6504-3:1998)
    Non-Patent Literature 2: JIS K 5906:1998

SUMMARY OF THE INVENTION

TECHNICAL PROBLEM

However, in the method in Non-Patent Literature 1, coated paper using dedicated hiding power test paper is required.

The method in Non-Patent Literature 2 is not applicable to pigment which does not float on water. In addition, the method in Non-Patent Literature 2 takes labor.

In the conventional technology for adjustment of the hiding power, it is required to prepare a paint preparation method for each of individual kinds of pigment, thereby resulting in inefficiency. In addition, it is required to make investigation as to a paint preparation method for new pigment.

As to a color tone, there are various measurement methods such as Hunter 1948 (L, a, b) color space, CIE 1976 (L*, a*, b*) color space, and Gloss, and though comparison with standard values is possible, as to the hiding power, relative evaluation by comparison with standard samples is still mainstream.

The present invention can reduce the time required for evaluation and a used paint amount when absolute evaluation and relative evaluation of the hiding power can be made by measuring visible light transmission intensity of a color card or coated paper, which is made available for color tone evaluation to confirm quality, such as art paper (coated paper) or PET film without using black and white hiding power test paper.

Therefore, an object of the present invention is to provide an simpler method for evaluating the hiding power.

SOLUTION TO PROBLEM

In order to solve the above-mentioned problem, the present inventors have devoted themselves to earnest research. As a result, as described in Examples in detail, the present inventors found that absorbance obtained on the basis of a ratio of light transmission amounts of coating film which contains pigment and coating film which contains no pigment is in a proportional relationship with a water surface diffusion area (WCA). In other words, according to the present invention, without using special test paper as a base material, only by measuring absorbance of coating film, hiding power of the coating film can be evaluated. In addition, according to the present invention, hiding power of even pigment whose WCA cannot be obtained and which does not flow on water can be evaluated by obtaining a value proportional to the WCA. Furthermore, it was found that the evaluation method of the present invention can evaluate hiding power not only in a case where the pigment is contained in the coating film formed on the base material but also in a case where the pigment is kneaded in the base material. Moreover, it was also found that by measuring absorbance of electromagnetic waves other than visible light, shielding power of the electromagnetic waves can be evaluated. On the basis of the findings obtained by the present inventors, a method for evaluating hiding power, a method for controlling quality, and a method for assuring quality, according to the present invention, have the below-described configurations.

The method for evaluating hiding power according to the present invention is a method for evaluating hiding power of a particle containing body which contains particles of a hiding power evaluation target, the method including a step of calculating, on a basis of a transmission amount $(I)$ of electromagnetic waves of the particle containing body and a base material and a transmission amount $(I_0)$ of electromagnetic waves of a non-particle containing body and a base material, absorbance (Abs) of electromagnetic waves by a below-mentioned Equation (1), the non-particle containing body having same composition as composition of the particle containing body except that the non-particle containing body does not contain the particles of the hiding power evaluation target,

$$\text{Equation (1)} \quad Abs = -\log(I/I_0).$$

Thus, it is not required to use hiding power test paper as a base material. **In** addition, a simpler method for evaluating hiding power can be provided.

Note that hereinafter, there may be a case where when electromagnetic waves are visible light, the absorbance (Abs) of the electromagnetic waves may be referred to as light absorbance (Abs).

It is preferable that the method for evaluating hiding power according to the present invention includes a step of calculating a value of (the absorbance (Abs))/(a concentration (C) of the particles of the particle containing body).

It is preferable that the method for evaluating hiding power according to the present invention includes a step of calculating a thickness (L) of the particle containing body having desired hiding power on a basis of the absorbance (Abs) of electromagnetic waves of the particle containing body, a concentration (C) of the particles, and an absorption coefficient $(\varepsilon)$ of electromagnetic waves of the particles by a below-mentioned Equation (2),

$$\text{Equation (2)} \quad L = Abs/(\varepsilon \times C).$$

It is preferable that the method for evaluating hiding power according to the present invention includes a step of calculating a concentration (C) of the particles of the particle containing body having desired hiding power on a basis of the absorbance (Abs) of the electromagnetic waves of the particle containing body, a thickness (L) of the particle containing body, and an absorption coefficient $(\varepsilon)$ of electromagnetic waves of the particles by a below-mentioned Equation (3),

$$\text{Equation (3)} \quad C = Abs/(\varepsilon \times L).$$

It is preferable that the method for evaluating hiding power according to the present invention includes a step of, on a basis of absorbance (Abs) of electromagnetic waves of the particle containing body having desired hiding power, absorbance $(Abs_1)$ of electromagnetic waves of a first particle containing body which contains only first particles as particles, and absorbance $(Abs_2)$ of electromagnetic waves of a second particle containing body which contains only second particles as particles, calculating a weight ratio (X) of the first particles and a weight ratio (1-X) of the second particles in a mixed particle containing body, having the desired hiding power, which contains the first particles and the second particles by a below-mentioned Equation (4),

$$\text{Equation (4)} \quad Abs_1 \times X + Abs_2 \times (1-X) = Abs \ (0 \leqq X \leqq 1).$$

It is preferable that in the method for evaluating hiding power according to the present invention, the particles are contained in coating film formed on the base material or are kneaded in the base material.

It is preferable that in the method for evaluating hiding power according to the present invention, the particles are pigment.

It is preferable that in the method for evaluating hiding power according to the present invention, the electromagnetic waves are visible light.

It is preferable that in the method for evaluating hiding power according to the present invention, the particles are particles having electromagnetic wave shielding performance and the hiding power is electromagnetic wave shielding power.

It is preferable that in the method for evaluating hiding power according to the present invention, the electromagnetic waves are electromagnetic waves other than visible light and the hiding power is electromagnetic wave shielding power.

A method for controlling quality of a product includes a step of prescribing absolute hiding power or relative hiding power by the above-mentioned method for evaluating hiding power and a step of performing quality control of a product on a basis of the absolute hiding power or the relative hiding power.

A method for assuring quality of a product includes a step of prescribing absolute hiding power and relative hiding power by the above-mentioned method for evaluating hiding power and a step of performing quality assurance of a product on a basis of the absolute hiding power or the relative hiding power.

BRIEF DESCRIPTION OF THE DRAWINGS

FIG. 1 is a graph showing relationship between absorbance (Abs) and a pigment concentration (C) of pigment containing coating film in each of Examples 1 to 7.
FIG. 2 is a graph showing relationship between values, each of which is obtained by diving the absorbance (Abs) of the pigment containing coating film in each of Examples 1 to 7 by each of the pigment concentrations (C), and water surface diffusion areas (WCA) of each pigment.
FIG. 3 is a graph showing relationship between absorbance (Abs) and a pigment concentration (C) of pigment containing coating film in each of Examples 8 to 14.
FIG. 4 is a graph showing relationship between values, each of which is obtained by diving the absorbance (Abs) of the pigment containing coating film in each of Examples 8 to 14 by each of the pigment concentrations (C), and water surface diffusion areas (WCA) of each pigment.
FIG. 5 is a graph showing relationship between absorbance (Abs) and a pigment concentration (C) of pigment containing coating film in each of Examples 15 to 21.
FIG. 6 is a graph showing relationship between values, each of which is obtained by diving the absorbance (Abs) of the pigment containing coating film in each of Examples 15 to 21 by each of the pigment concentrations (C), and water surface diffusion areas (WCA) of each pigment.
FIG. 7 is a graph showing relationship between absorbance (Abs) and a pigment concentration (C) of pigment containing coating film in each of Examples 23 to 24.

DESCRIPTION OF EMBODIMENT

Hereinafter, with reference to specific examples, a method for evaluating hiding power according to the present invention will be described in detail. Note that the present invention is not limited to the below-described embodiment and a variety of modifications can be made without departing from the technical idea of the present invention.

The method for evaluating hiding power according to the present invention is a method for evaluating hiding power of a particle containing body which contains particles as a hiding power evaluation target, the method including a step of calculating absorbance (Abs) of electromagnetic waves on the basis of a transmission amount (I) of electromagnetic waves of the particle containing body and a base material and a transmission amount ($I_0$) of electromagnetic waves of a non-particle containing body and a base material, the non-particle containing body having the same composition as that of the particle containing body except that the non-particle containing body contains no particles, by the following Equation (1).

$$\text{Equation (1)} \quad \text{Abs} = -\log(I/I_0)$$

As one embodiment, a method for evaluating hiding power in a case where the hiding power evaluation target is coating film which contains pigment and hiding power of visible light is evaluated will be described.

First, paint which contains particles of pigment is applied onto a base material (hereinafter, also referred to as "undercoat") and is dried, thereby forming pigment containing coating film. Next, the pigment non-containing coating film (hereinafter, also referred to as a "blank" or "control") having the same composition as that of the pigment containing coating film except that the pigment non-containing coating film contains no particles of the pigment is formed onto a base material or undercoat. Note that the pigment containing coating film is one example of the particle containing body and the pigment non-containing coating film is one example of the non-particle containing body.

As the paint which contains the pigment, for example, the heretofore known paint such as paint obtained by dispersing aluminum pigment (paste) in nitrocellulose (cellulose nitrate) lacquer, acryl lacquer, or waterborne paint is used.

Since due to affinity of a resin component and the pigment in the paint, the later-descried correlation coefficient of Abs and WCA fluctuates, it is desired that the nitrocellulose lacquer having a wide range of affinity is used.

Th base material is not limited and as the base material, a color card, coated paper, or the like, which is made available for color tone evaluation to confirm quality, such as art paper (coated paper) and PET film can be used.

A method for preparing the coating film is made the same between the pigment containing coating film and the pigment non-containing coating film, and conditions of the lacquer, a film thickness of the coating film, the color card, an applicating method, a drying temperature, a drying method, and the like are made the same therebetween.

Paint application is made, for example, by employing a doctor blade method and thereby applying the paint onto the base material. For example, the commercially available nitrocellulose (cellulose nitrate) lacquer is used, the doctor blade method is employed, and application onto the PET film or the art paper (coated paper) is made, thereby allowing the particle containing body and a particle non-containing body to be obtained.

As a film applicator used in the doctor blade method, in order to make orientation of the pigment constant, for example, a film applicator having 2 mils (50.8 $\mu$m) can be used, and a film applicator having 6 mils, 9 mils, or the like may be used. A thickness of the coating film obtained by the application in 9 mils is 4.5-fold of that of coating film obtained by the application in 2 mils, and there is a case where due to disorder of orientation of pigment and influence of secondary reflection, absorbance Abs becomes slightly smaller than the 4.5-fold.

The pigment containing coating film obtained as described above contains particles of pigment. Besides the coating film, the particle containing body may be prepared by kneading particles of pigment or the like in the base material.

As to the obtained particle containing body (pigment containing coating film) and particle non-containing body (pigment non-containing coating film), transmission amounts I and $I_0$ of light of wavelengths for evaluating the hiding power are measured and on the basis of Equation (1) Abs = -log($I/I_0$), absorbance (Abs) is obtained. It is only required for the transmission amounts I and $I_0$ of light to be measured by the heretofore known method allowing the transmission amounts of light of wavelengths, for evaluating the hiding power, to be measured, the method is not limited, and as one example, a spectrophotometric colorimeter CM-5 (Konica Minolta, Inc.) can be used. For transmittance measurement, it is required that light intensity is sufficiently strong or detection capability of a detection device which detects light passing through the coating film is sufficiently high. In a case where the spectrophotometric colorimeter is used, brightness (L* value) can be treated as a transmission amount of light.

When hiding power of visible light rays is evaluated, in a case where a base material or undercoat is opaque to the visible light rays, there may be a case where the transmittance measurement cannot be made by the spectrophotometric colorimeter CM-5. In such a case, the transmittance may be measured by using a white LED light source and an illuminometer.

In a case such as a case where as particles, particles having electromagnetic wave shielding performance are used, hiding power as to electromagnetic waves other than the visible light rays can also be evaluated. A wavelength of the electromagnetic waves for evaluating the hiding power is not limited. For example, those of long waves, medium waves, short waves, ultrashort waves, microwaves, radio waves, infrared rays, visible light rays, ultraviolet rays, EUV, X rays, and gamma rays are also applicable. Also in a case where hiding power of the electromagnetic waves other than the visible light is evaluated, on the basis of Equation (1) Abs = -log($I/I_0$), absorbance (Abs) of electromagnetic waves of a wavelength for evaluating hiding power is obtained. It is only required for measurement of transmission amounts of the electromagnetic waves to select a light source which is capable of radiating electromagnetic waves each having a required wavelength and an electromagnetic wave detector which deals with that wavelength.

A value of the absorbance (Abs) obtained as described above reflects hiding power of particles of pigment or the like as described in Examples in detail.

Thus, using the hiding power test paper as the base material is not required. **In** addition, a simpler method for evaluating the hiding power can be provided.

Furthermore, it is preferable that the method includes a step of calculating a value of (absorbance (Abs))/(concentration (C) of particles of a particle containing body). The present inventors found that the value of (absorbance (Abs))/(concentration (C) of particles of a particle containing body) is proportional to a WCA (water surface diffusion area). Accordingly, by calculating the value of (absorbance (Abs))/(concentration (C) of particles of a particle containing body), also as to particles which do not flow on water, hiding power of such particles can be evaluated without measuring the WCA as in a case where the WCA is used.

The relationship of a thickness (L) of a particle containing body (pigment containing coating film), a absorption coefficient ($\varepsilon$) of electromagnetic waves (visible light rays) of particles (pigment), and a concentration (C) of the particles (pigment) in the particle containing body (pigment containing coating film) is absorbance (Abs) = $\varepsilon \times L \times C$ (Beer-Lambert-Bouguer law). Therefore, a thickness (L) of a particle containing body having desired hiding power can be calculated on the basis of the absorbance (Abs) of the electromagnetic waves of the particle containing body, the concentration (C) of the particles, and the absorption coefficient ($\varepsilon$) of the electromagnetic waves of the particles by the following Equation (2).

$$\text{Equation (2) } L = \text{Abs}/(\varepsilon \times C)$$

In addition, a concentration (C) of the particles of the particle containing body having the desired hiding power can be calculated on the basis of the absorbance (Abs) of the electromagnetic waves of the particle containing body, the thickness (L) of the particle containing body, and the absorption coefficient ($\varepsilon$) of the electromagnetic waves of the particles by the following Equation (3).

$$\text{Equation (3) } C = \text{Abs}/(\varepsilon \times L)$$

In the method for evaluating hiding power according to the present invention, as to a mixed particle containing body in which two kinds of particles are mixed, a weight ratio of the respective kinds of particles required for obtaining desired hiding power can be obtained. In other words, on the basis of absorbance (Abs) of electromagnetic waves of a particle containing body having desired hiding power, absorbance ($\text{Abs}_1$) of electromagnetic waves of a first particle containing body which contains only first particles as particles, and absorbance ($\text{Abs}_2$) of electromagnetic waves of a second particle containing body which contains only second particles as particles, a weight ratio (X) of the first particles and a weight ratio (1-X) of the second particles in the mixed particle containing body which has the desired hiding power and contains the first particles and the second particles can be calculated by the following Equation (4).

$$\text{Equation (4) } \text{Abs}_1 \times X + \text{Abs}_2 \times (1-X) = \text{Abs} \ (0 \leqq X \leqq 1)$$

A method for controlling quality according to the present invention includes a step of prescribing absolute hiding power or relative hiding power by the above-described method for evaluating hiding power and a step of controlling quality of a product on the basis of the absolute hiding power or the relative hiding power.
A method for assuring quality according to the present invention includes a step of prescribing absolute hiding power and relative hiding power by the above-described method for evaluating hiding power and a step of assuring quality of a product on the basis of the absolute hiding power or the relative hiding power.

[Examples]

Hereinafter, with reference to specific examples, a method for evaluating hiding power according to the present invention will be described in detail. Note that the present invention is not limited to the below-described embodiment and a variety of modifications can be made without departing from the technical idea of the present invention.

(Examples 1 to 7)

As the pigment, kinds of aluminum pigment (paste) A to G shown in Table 1 were used, paste weights thereof shown in a to c in Table 2 were weighed, and commercially available nitrocellulose lacquer was added thereto so as to achieve 50 g of a total weight. Then, uniform agitation was performed by a planetary mixer, thereby preparing each paint. Each of the average particle diameters $D_{50}$ was measured by a particle diameter distribution measurement apparatus MT3300EX II (MicrotracBEL Corp.). The WCA of each pigment was measured on the basis of Non-Patent Literature 2.

[Table 1]

| | Solid Content | Average Particle Diameter $D_{50}$ ($\mu$m) | WCA ($cm^2$/g) |
|---|---|---|---|
| Aluminum Pigment A | 70.0% | 10 | 24590 |
| Aluminum Pigment B | 41.1% | 11 | 53769 |
| Aluminum Pigment C | 74.1% | 25 | 8667 |
| Aluminum Pigment D | 70.1% | 13 | 29756 |
| Aluminum Pigment E | 66.3% | 9 | 38781 |
| Aluminum Pigment F | 65.0% | 23 | 14327 |
| Aluminum Pigment G | 71.2% | 33 | 4813 |
| Aluminum Pigment H | 70.0% | 9 | 32427 |
| Aluminum Pigment I | 71.0% | 14 | 16463 |

[Table 2]

| | Pigment | a | | b | | c | |
|---|---|---|---|---|---|---|---|
| | | Paste Weight in 50 g of Paint | C | Paste Weight in 50 g of Paint | C | Paste Weight in 50 g of Paint | C |
| Example 1 | Aluminum Pigment A | 1.5g | 2.10% | 2.0g | 2.80% | 2.5g | 3.50% |
| Example 2 | Aluminum Pigment B | 1.0g | 0.82% | 1.5g | 1.23% | 2.0g | 1.64% |
| Example 3 | Aluminum Pigment C | 1.5g | 2.22% | 3.0g | 4.45% | 4.5g | 6.67% |
| Example 4 | Aluminum Pigment D | 1.0g | 1.40% | 1.5g | 2.10% | 2.0g | 2.80% |
| Example 5 | Aluminum Pigment E | 0.5g | 0.66% | 1.0g | 1.33% | 1.5g | 1.99% |
| Example 6 | Aluminum Pigment F | 1.5g | 1.95% | 3.0g | 3.90% | 4.5g | 5.85% |
| Example 7 | Aluminum Pigment G | 1.5g | 2.13% | 3.0g | 4.27% | 4.5g | 6.40% |

Obtained each paint was applied onto PET film by using an applicator having 2 mils (50.8 $\mu$m), and the resultant was dried at a room temperature, thereby obtaining pigment containing coating film in each of Examples. As a blank, nitrocellulose lacquer was applied thereonto by using an applicator having 2 mils, and the resultant was dried at a room temperature, thereby preparing pigment non-containing coating film.

Each pigment containing coating film and each pigment non-containing coating film, prepared on the PET film, were cut together with the PET film in six-cmbysix-cm square pieces, thereby obtaining test specimens. An L* value of the test specimen of the pigment containing coating film and an L* value of the test specimen of the pigment non-containing coating film were measured by using a transmitted light intensity measurement unit of a spectrophotometric colorimeter CM-5 (manufactured by Konica Minolta, Inc.), and (L* value of pigment containing coating film)/(L* value of pigment non-containing coating film) was treated as transmittance, thereby obtaining absorbance Abs. A result is shown in Table 3.

[Table 3]

| | Abs | | | Abs/C | WCA/(Abs/C) |
|---|---|---|---|---|---|
| | a | b | c | | |
| Example 1 | 0.329 | 0.459 | 0.592 | 16.52 | 1489 |
| Example 2 | 0.256 | 0.401 | 0.563 | 33.35 | 1612 |
| Example 3 | 0.108 | 0.231 | 0.378 | 5.47 | 1584 |
| Example 4 | 0.235 | 0.372 | 0.530 | 18.24 | 1631 |
| Example 5 | 0.167 | 0.359 | 0.561 | 27.65 | 1402 |
| Example 6 | 0.155 | 0.333 | 0.548 | 9.03 | 1587 |
| Example 7 | 0.062 | 0.129 | 0.198 | 3.06 | 1574 |

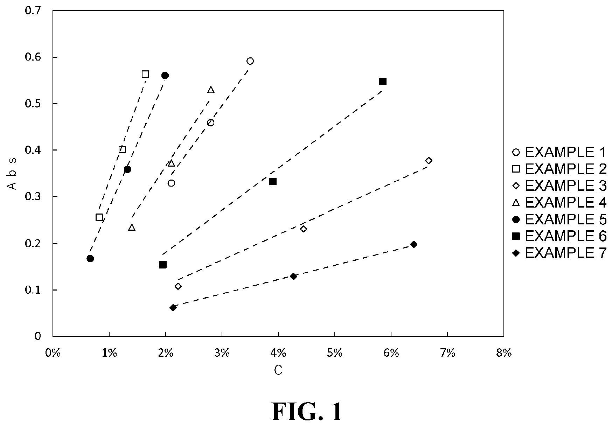

Each absorbance Abs plotted with respect to the pigment concentration C on the basis of this result is shown in FIG. 1. It is seen from FIG. 1 that as to any of kinds of pigment, the relationship of the absorbance Abs and the pigment concentration C can be approximated by a straight line passing through a point of origin. In addition, values of inclination Abs/C of these straight lines passing through the point of origin, obtained by the least squares method, are shown in Table 3.

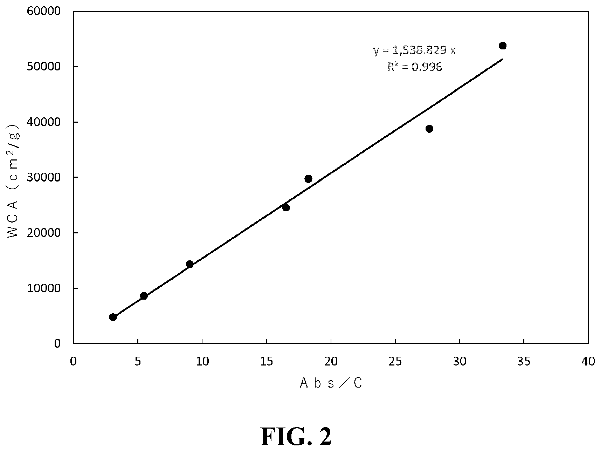

The WCAs of the respective kinds of pigment plotted with respect to the above-mentioned Abs/C are shown in FIG. 2. The inclination obtained by the least squares method was 1538.8. From this result, Equation (5) can be derived.

It is seen from FIG. 2 that since as to metal effect pigment, a ratio of Abs/C is proportional to WCA, absorbance Abs reflects hiding power.

(Examples 8 to 14)

In a manner similar to the manner in which the paint in Example 1 was prepared, respective kinds of paint of aluminum pigment (paste) A to G whose pigment concentrations C were shown in Table 4 were prepared. Each of the respective kinds of paint was applied to art paper by using an applicator having 2 mils (50.8 μm) and the resultant was dried at a room temperature, thereby obtaining pigment containing coating film in each of Examples. In addition, nitrocellulose lacquer used for preparation of the paint was separately applied to art paper as it was by using an applicator having 2 mils and the resultant was dried at a room temperature, thereby preparing pigment non-containing coating film.

Each of the prepared kinds of pigment containing coating film and the pigment non-containing coating film were irradiated with a white LED whose front illuminance was 345,000 lux and by using the commercially available illuminometer, values of light transmission amounts I of respective specimens of the coating film and a light transmission amount $I_0$ of standard coating film were measured. From the light source to the illuminometer, in order to avoid leak and mixing of light from an external light source, cylindrical black ABS resin was used. The apparatus was configured such that a distance from the light source to the coating film was approximately 40 mm and a distance from the coating film to a surface of the illuminometer was approximately 15 mm. The absorbance Abs of the specimens of coating film, calculated from the measurement result, are shown in Table 4.

[Table 4]

| Example 8 | Pigment A | C | 2.10% | 2.80% | 4.20% | | | |
|---|---|---|---|---|---|---|---|---|
| | | Abs | 0.673 | 0.930 | 1.369 | | | |
| Example 9 | Pigment B | C | 1.23% | 1.64% | 2.46% | 3.28% | 4.11% | 4.93% |
| | | Abs | 0.810 | 1.109 | 1.643 | 2.246 | 2.863 | 3.514 |
| Example 10 | Pigment C | C | 2.22% | 2.96% | 4.45% | | | |
| | | Abs | 0.243 | 0.334 | 0.501 | | | |
| Example 11 | Pigment D | C | 2.10% | 2.80% | 4.20% | | | |
| | | Abs | 0.755 | 1.035 | 1.525 | | | |
| Example 12 | Pigment E | C | 1.99% | 2.65% | 3.98% | | | |
| | | Abs | 1.012 | 1.320 | 2.002 | | | |
| Example 13 | Pigment F | C | 1.95% | 2.60% | 3.90% | | | |
| | | Abs | 0.350 | 0.474 | 0.705 | | | |
| Example 14 | Pigment G | C | 2.13% | 2.85% | 4.27% | | | |
| | | Abs | 0.130 | 0.180 | 0.269 | | | |

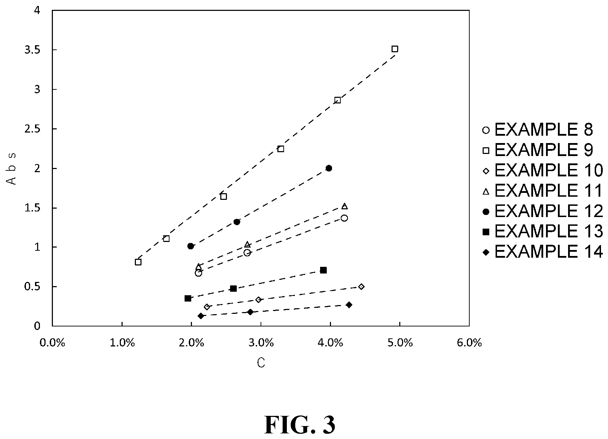

On the basis of this result, the absorbance Abs thereof plotted with respect to the pigment concentrations C is shown in FIG. 3. It is seen from FIG. 3 that also as to any of kinds of the coating film using the art paper, the relationship of the absorbance Abs and the pigment concentrations C of any of the pigment can be approximated by straight lines passing through a point of origin. In addition, values of inclination Abs/C of these straight lines passing through a point of origin, obtained by the least squares method, are shown in Table 5.

[Table 5]

| | WCA (cm²/g) | Abs/C | WCA/(Abs/C) |
|---|---|---|---|
| Example 8 | 24590 | 32.68 | 752 |
| Example 9 | 53769 | 69.64 | 772 |
| Example 10 | 8667 | 11.21 | 773 |
| Example 11 | 29756 | 36.38 | 818 |
| Example 12 | 38781 | 50.26 | 772 |
| Example 13 | 14327 | 18.11 | 791 |
| Example 14 | 4813 | 6.269 | 768 |

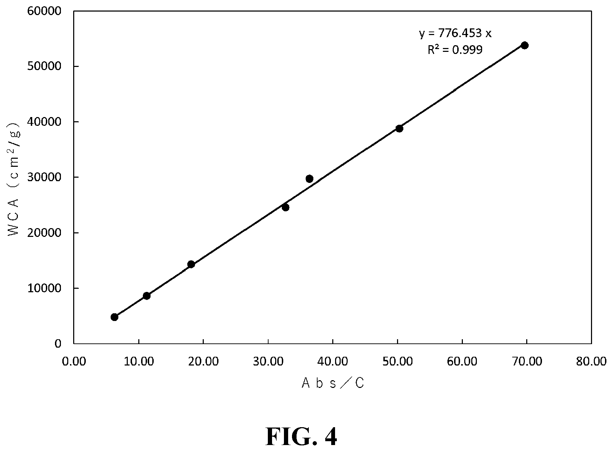

The WCAs of respective kinds of pigment plotted with respect to the above-mentioned Abs/C are shown in FIG. 4. It is from FIG. 4 that even in a case where as a base material, opaque art paper is used, a ratio of Abs/C is proportional to WCA, hiding power can be evaluated. The inclination obtained by the least squares method was 776.5. From this result, Equation (6) can be derived.

(Examples 15 to 21)

Each of kinds of paint of pigment was adjusted so as to achieve each pigment concentration C shown in Table 6. In a manner similar to the manner in which the measurement of absorbance Abs in Example 8 was made except that an applicator having 9 mils (228.6 $\mu$m) was used, by using each of the obtained kinds of paint, measurement of absorbance Abs was made. A result is shown in Table 6.

[Table 6]

| | | | | | | | | | | |
|---|---|---|---|---|---|---|---|---|---|---|
| Example 15 | Pigment A | c | 2.10% | 0.98% | 1.68% | 1.40% | 0.42% | 0.14% | 2.52% | 0.70% |
| | | Abs | 2.478 | 1.158 | 1.989 | 1.647 | 0.487 | 0.171 | 3.008 | 0.831 |
| Example 16 | Pigment B | c | 1.68% | 1.40% | 0.70% | 0.14% | 0.98% | 0.42% | | |
| | | Abs | 3.835 | 3.242 | 1.681 | 0.354 | 2.245 | 1.017 | | |
| Example 17 | Pigment C | c | 1.39% | 0.70% | 2.52% | 0.13% | | | | |
| | | Abs | 0.637 | 0.327 | 1.128 | 0.081 | | | | |
| Example 18 | Pigment D | c | 1.40% | 0.70% | 2.52% | 0.14% | | | | |
| | | Abs | 1.831 | 0.986 | 3.447 | 0.210 | | | | |
| Example 19 | Pigment E | c | 1.41% | 0.70% | 2.52% | 0.15% | | | | |
| | | Abs | 2.565 | 1.297 | 4.193 | 0.338 | | | | |
| Example 20 | Pigment F | c | 1.40% | 0.70% | 2.52% | 0.14% | | | | |
| | | Abs | 0.975 | 0.506 | 1.704 | 0.097 | | | | |
| Example 21 | Pigment G | c | 1.39% | 0.70% | 2.52% | 0.14% | | | | |
| | | Abs | 0.380 | 0.186 | 0.710 | 0.046 | | | | |

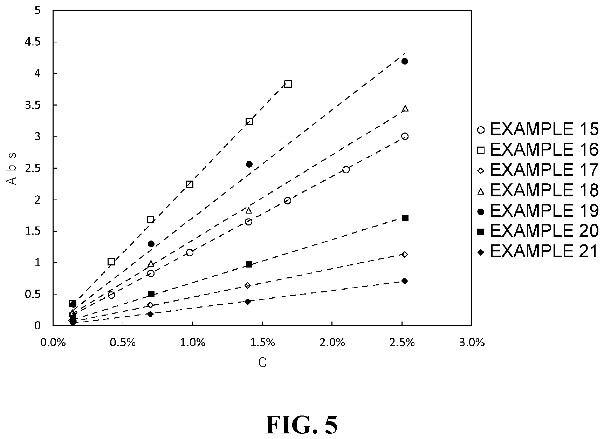

On the basis of this result, the absorbance Abs plotted with respect to the pigment concentrations C is shown in FIG. 5. It is seen from FIG. 5 that also as to any of kinds of the coating film using the art paper, the relationship of the absorbance Abs and the pigment concentrations C of any kinds of the pigment can be approximated by straight lines passing through a point of origin. In addition, values of inclination Abs/C of these straight lines passing through a point of origin, obtained by the least squares method, are shown in Table 7.

[Table 7]

| | WCA (cm$^2$/g) | Abs/C | WCA/(Abs/C) |
|---|---|---|---|
| Example 15 | 24590 | 118.54 | 207 |
| Example 16 | 53769 | 230.55 | 233 |
| Example 17 | 8667 | 45.14 | 192 |
| Example 18 | 29756 | 135.56 | 220 |
| Example 19 | 38781 | 171.20 | 227 |
| Example 20 | 14327 | 68.25 | 210 |
| Example 21 | 4813 | 27.923 | 172 |

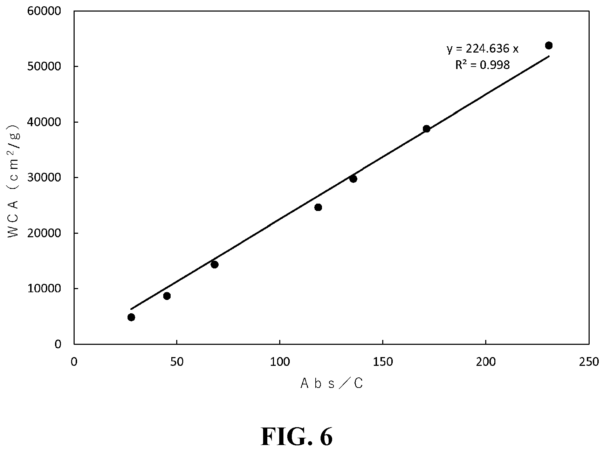

The WCAs of respective kinds of pigment plotted with respect to the above-mentioned Abs/C are shown in FIG. 6. The inclination obtained by the least squares method was 224.6. From this result, Equation (7) can be derived.

It is seen from FIG. 6 that even in a case where an application amount of the paint is changed, as long as the measurement was made under the same conditions, the hiding power can be evaluated.

$$\text{Equation (5) WCA} = 1538.8 \times \text{Abs/C}$$

(in a case where as in Example 1 to 7, the test coating film was prepared and the transmittance was measured)

$$\text{Equation (6)} \quad WCA = 776.5 \times Abs/C$$

(in a case where as in Examples 8 to 14, the test coating film was prepared and the transmittance was measured)

$$\text{Equation (7)} \quad WCA = 224.6 \times Abs/C$$

(in a case where as in Examples 15 to 21, the test coating film was prepared and the transmittance was measured)

As described above, even as to hydrophilic pigment whose WCA cannot be obtained by the method in Non-Patent Literature 2, by forming coating film onto PET film or art paper and actually measuring absorbance Abs and a pigment concentration C, an estimated WCA can be obtained from the above-described correlation coefficient. Specifically, as an estimated WCA value of aluminum pigment EMR-B5680 (paste manufactured by TOYO ALUMINIUM K.K.) whose WCA cannot be obtained by the method in Non-Patent Literature 2 and which was coated with silica, 24,000 cm$^2$/g (per one g of aluminum) was obtained. The preparation of the paint-coating film and the measurement of absorbance Abs were made by the method in each of Examples 15 to 21 (the obtained absorbance Abs and Abs/C as shown in Table 8) and by using Equation (7), the estimated WCA value was calculated (the estimated WCA = 224.6 $\times$ 106.7 = 24,000 cm$^2$/g).

[Table 8]

|  | C | Abs | Abs/C |
|---|---|---|---|
| EMR-B5680 | 0.70% | 0.747 | 106.7 |

(Example 22)

(Adjustment of Hiding Power of Pigment)

Specimen pigment having absorbance Abs in a pigment concentration C is used and from absorbance $Abs_0$ in a pigment concentration $C_0$ of target pigment having desired hiding power, by pigment concentration $C' = C \times Abs_0/Abs$ imparting hiding power equivalent to hiding power in the concentration $C_0$ of the target pigment, the specimen pigment is used and paint having hiding power equivalent to that of the target pigment can be prepared ($C_0$ and C may be equal to or differed from each other).

As pigment, kinds of aluminum pigment (paste) A, H, and I shown in Table 9 were used, each paste was weighed so as to achieve 0.7 g of a weight of the pigment, and commercially available nitrocellulose lacquer was added thereto so as to achieve 50 g of a total weight. Then, uniform agitation was performed by a planetary mixer, thereby preparing each paint. The prepared each paint was applied to art paper by using an applicator having 9 mils (228.6 μm) and the resultant was dried at a room temperature, thereby obtaining each specimen coating film. In addition, nitrocellulose lacquer used for preparation of the paint was separately applied to art paper as it was by using an applicator having 9 mils and the resultant was dried at a room temperature, thereby preparing target coating film having desired hiding power. Absorbance Abs of each of the kinds of aluminum pigment A, H, and I was obtained by a measurement method similar to that employed in Example 15 and relative absorbance with respect to absorbance Abs of target pigment was calculated. The relative absorbance is shown in Table 9.

[Table 9]

|  |  | Solid Content | Relative Absorbance | Pigment Weight Ratio |
|---|---|---|---|---|
| Target Pigment | Aluminum Pigment A | 70% | 1.00 |  |
| First Pigment | Aluminum Pigment H | 70% | 1.34 | 48.0% |
| Second Pigment | Aluminum Pigment I | 71% | 0.69 | 52.0% |
| Example 22 Mixed Pigment | Calculated value | 70% | 1.00 |  |
|  | Actually Measured Value | 70% | 1.02 |  |

When a weight ratio of the aluminum pigment H is defined as X and a weight ratio of the aluminum pigment I is defined as (1-X), the following Equation (8) can be established.

Equation (8) (relative absorbance of the coating film containing the pigment H with respect to the target coating film)$\times$X+(relative absorbance of the coating film containing the pigment I with respect to the target coating film)$\times$(1-X) = 1(0$\leqq$x$\leqq$1)

In a case of Example 22,

$$1.34\times X+0.69\times(1\text{-}X) \;=\; 1.00$$

$$X \;=\; 0.48$$

On the basis of a pigment weight ratio calculated by Equation (8), the aluminum pigment (paste) H and the aluminum pigment (paste) I were mixed, thereby preparing mixed pigment. The obtained mixed pigment was collected so as to achieve 0.7 g of a total weight of the pigment blended into the paint and the commercially available nitrocellulose lacquer was added thereto so as to achieve 50 g of a total amount. Then, uniform agitation was performed by a planetary mixer, thereby preparing the paint. The prepared paint was applied to art paper by using an applicator having 9 mils (228.6 $\mu$m) and the resultant was dried at a room temperature, thereby obtaining specimen coating film. In addition, nitrocellulose lacquer used for preparation of the paint was separately applied to art paper as it was by using an applicator having 9 mils and the resultant was dried at a room temperature, thereby preparing pigment non-containing coating film.

Absorbance Abs of the mixed pigment (paste) was obtained by a measurement method similar to that employed in Example 15, relative absorbance with respect to the absorbance Abs of the target pigment was calculated, and as a result, a value of the relative absorbance with respect to that of the target pigment A was 1.02, whereby it was made possible to confirm that the mixed pigment had hiding power equivalent to that of the target pigment. Thus, it is made possible to suppress variation of hiding power caused, for example, among production lots of pigment.

(Example 23)

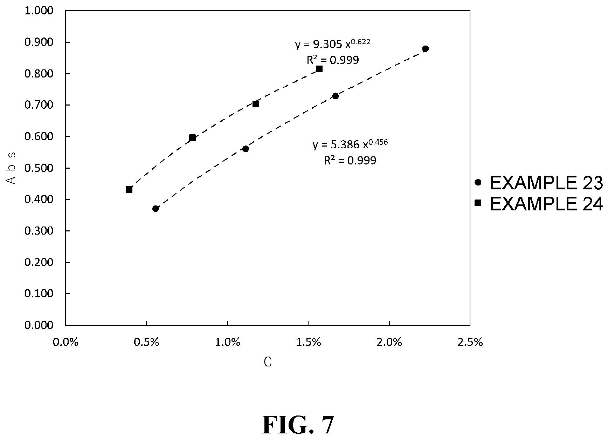

As pigment, 30.0 kg of blue pigment shown in Table 10, 75.0 kg of HAWS, and 3.0 kg of a dispersing agent were crushed by a bead mill, thereby obtaining slurry having 27.8% of a pigment concentration. By using the slurry obtained instead of the aluminum pigment, absorbance Abs at a pigment concentration C in each paint shown in Table 10 was measured in a manner similar to the manner in Example 8. The result is shown in Table 10 and FIG. 7.

[Table 10]

| | | | | | | |
|---|---|---|---|---|---|---|
| Example 23 | Blue Pigment Lionol Blue 7185-PM | C | 0.56% | 1.11% | 1.67% | 2.22% |
| | | Abs | 0.371 | 0.560 | 0.728 | 0.879 |
| Example 24 | Red Pigment Irgazin Red L3630 | C | 0.39% | 0.78% | 1.18% | 1.57% |
| | | Abs | 0.431 | 0.596 | 0.703 | 0.815 |

(Example 24)

As pigment, 4.6 kg of red pigment shown in Table 10, 25.1 kg of HAWS, and 0.5 kg of a dispersing agent were crushed by a bead mill, thereby obtaining slurry. By using the obtained slurry, absorbance Abs was measured in a manner similar to the manner in Example 23. The result is shown in Table 10 and FIG. 7.

It is seen from FIG. 7 that although as to chromatic colored pigment, a value of Abs/C is not constant, a calibration curve which can be approximated can be made.

The obtained calibration curve can be used for adjustment of the hiding power as with examination using the aluminum pigment.

The described embodiment and Examples are to be considered in all respects only as illustrative and not restrictive. It is intended that the scope of the invention is, therefore, indicated by the appended claims rather than the foregoing description of the embodiment and Examples and that all modifications and variations coming within the meaning and equivalency range of the appended claims are embraced within their scope.

**Claims**

1. A method for evaluating hiding power of a particle containing body which contains particles of a hiding power evaluation target, the method including
a step of calculating, on a basis of a transmission amount (I) of electromagnetic waves of the particle containing body and a base material and a transmission amount ($I_0$) of electromagnetic waves of a non-particle containing body and a base material, absorbance (Abs) of electromagnetic waves by a below-mentioned Equation (1), the non-particle containing body having same composition as composition of the particle containing body except that the non-particle containing body does not contain the particles of the hiding power evaluation target,

$$\text{Equation (1) } Abs = -\log(I/I_0).$$

2. The method for evaluating hiding power according to claim 1, the method including a step of calculating a value of (the absorbance (Abs))/(a concentration (C) of the particles of the particle containing body).

3. The method for evaluating hiding power according to claim 1, the method including a step of calculating a thickness (L) of the particle containing body having desired hiding power on a basis of the absorbance (Abs) of the electromagnetic waves of the particle containing body, a concentration (C) of the particles, and an absorption coefficient ($\varepsilon$) of electromagnetic waves of the particles by a below-mentioned Equation (2),

$$\text{Equation (2) } L = Abs/(\varepsilon \times C)$$

4. The method for evaluating hiding power according to claim 1, the method including a step of calculating a concentration (C) of the particles of the particle containing body having desired hiding power on a basis of the absorbance (Abs) of the electromagnetic waves of the particle containing body, a thickness (L) of the particle containing body, and an absorption coefficient ($\varepsilon$) of electromagnetic waves of the particles by a below-mentioned Equation (3),

$$\text{Equation (3) } C = Abs/(\varepsilon \times L)$$

5. The method for evaluating hiding power according to claim 1, the method including a step of, on a basis of absorbance (Abs) of electromagnetic waves of the particle containing body having desired hiding power, absorbance ($Abs_1$) of electromagnetic waves of a first particle containing body which contains only first particles as particles, and absorbance ($Abs_2$) of electromagnetic waves of a second particle containing body which contains only second particles as particles, calculating a weight ratio (X) of the first particles and a weight ratio (1-X) of the second particles in a mixed particle containing body, having the desired hiding power, which contains the first particles and the second particles by a below-mentioned Equation (4),

$$\text{Equation (4) } Abs_1 \times X + Abs_2 \times (1-X) = Abs \ (0 \leqq X \leqq 1).$$

6. The method for evaluating hiding power according to any one of claims 1 to 5, wherein the particles are contained in coating film being formed on the base material or are kneaded in the base material.

7. The method for evaluating hiding power according to claim 6, wherein the particles are pigment.

8. The method for evaluating hiding power according to claim 7, wherein the electromagnetic waves are visible light.

9. The method for evaluating hiding power according to claim 6, wherein the particles are particles having electromagnetic wave shielding performance and the hiding power is electromagnetic wave shielding power.

10. The method for evaluating hiding power according to claim 9, wherein the electromagnetic waves are electromagnetic waves other than visible light and the hiding power is electromagnetic wave shielding power.

11. A method for controlling quality of a product, the method including a step of prescribing absolute hiding power or relative hiding power by the method for evaluating hiding power according claim 6 and a step of performing quality control of a product on a basis of the absolute hiding power or the relative hiding power.

12. A method for assuring quality of a product, the method including a step of prescribing absolute hiding power and relative hiding power by the method for evaluating hiding power according to claim 6 and a step of performing quality assurance of a product on a basis of the absolute hiding power or the relative hiding power.

**FIG. 1**

**FIG. 2**

**FIG. 3**

FIG. 4

**FIG. 5**

**FIG. 6**

**FIG. 7**

## INTERNATIONAL SEARCH REPORT

| International application No. |
|---|
| **PCT/JP2023/025720** |

**A. CLASSIFICATION OF SUBJECT MATTER**

*G01N 21/59*(2006.01)i; *C09D 7/61*(2018.01)i; *C09D 201/00*(2006.01)i
FI:  G01N21/59 M; C09D201/00; C09D7/61

According to International Patent Classification (IPC) or to both national classification and IPC

**B. FIELDS SEARCHED**

Minimum documentation searched (classification system followed by classification symbols)

G01N21/00-21/61; G01N23/00-23/2276; C09D1/00-7/80; C09D17/00; C09D201/00-201/10; B05D1/00-7/26

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2015-184648 A (TOYO BOSEKI) 22 October 2015 (2015-10-22)<br>paragraphs [0007], [0018], [0054], [0057] | 1-12 |
| X | JP 2002-535674 A (E.I. DU PONT DE NEMOURS AND COMPANY) 22 October 2002 (2002-10-22)<br>paragraphs [0050]-[0056] | 1-12 |
| X | WO 2015/146977 A1 (TOYO ALUMINIUM KABUSHIKI KAISHA) 01 October 2015 (2015-10-01)<br>paragraphs [0031], [0101]-[0103] | 1-12 |
| X | JP 2019-167510 A (ASAHI KASEI CORP) 03 October 2019 (2019-10-03)<br>paragraphs [0045], [0047] | 1-12 |
| A | JP 2014-16214 A (NIPPON TESTPANEL CO LTD) 30 January 2014 (2014-01-30) | 1-12 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 September 2023** | **19 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/025720**

| Patent document cited in search report | | | Publication date (day/month/year) | Patent family member(s) | | | Publication date (day/month/year) |
|---|---|---|---|---|---|---|---|
| JP | 2015-184648 | A | 22 October 2015 | (Family: none) | | | |
| JP | 2002-535674 | A | 22 October 2002 | US | 6040913 | A | |
| | | | | column 8, line 11 to column 9, line 11 | | | |
| | | | | EP | 1147397 | A1 | |
| | | | | US | 6236460 | B1 | |
| | | | | WO | 00/45152 | A1 | |
| | | | | CA | 2360274 | A1 | |
| WO | 2015/146977 | A1 | 01 October 2015 | EP | 3124141 | A1 | |
| | | | | paragraphs [0031], [0101]-[0103] | | | |
| | | | | US | 2017/0001242 | A1 | |
| | | | | CN | 106029266 | A | |
| | | | | KR | 10-2016-0136302 | A | |
| JP | 2019-167510 | A | 03 October 2019 | (Family: none) | | | |
| JP | 2014-16214 | A | 30 January 2014 | JP | 5224562 | B1 | |

Form PCT/ISA/210 (patent family annex) (January 2015)